(19) Europäisches Patentamt
European Patent Office
Office européen des brevets

(11) Numéro de publication : **0 392 913 B1**

# (12) FASCICULE DE BREVET EUROPEEN

(45) Date de publication du fascicule du brevet : **23.06.93 Bulletin 93/25**

(51) Int. Cl.$^5$ : **C07D 471/14,** A61K 31/435, // (C07D471/14, 221:00, 221:00, 209:00)

(21) Numéro de dépôt : **90400968.5**

(22) Date de dépôt : **10.04.90**

## (54) Trichlorhydrate de (diéthylamino-3 propyl)-amino-1 méthyl-5 dipyrido [4,3-b] [3,4-f] indole.

(30) Priorité : **10.04.89 FR 8904689**

(43) Date de publication de la demande : **17.10.90 Bulletin 90/42**

(45) Mention de la délivrance du brevet : **23.06.93 Bulletin 93/25**

(84) Etats contractants désignés : **AT BE CH DE DK ES FR GB GR IT LI LU NL SE**

(56) Documents cités :
**CHEMICAL ABSTRACTS, vol. 102, 1985, page 37, résumé no. 72547f, Columbus, Ohio, US; M.J. VILAREM et al.: "Effects of BD-40, an ellipticine analog (aza-ellipticine) on cell cycle traverse and DNA synthesis in cultures of synchronized mouse fibroblasts"**
**JOURNAL OF LIQUID CHROMATOGRAPHY, vol. 6, no. 5, 1983, pages 939-949**

(73) Titulaire : **ELF SANOFI**
**32-34, rue Marbeuf**
**F-75008 Paris (FR)**

(72) Inventeur : **Bisagni, Emile**
**16, rue Bossuet**
**F-91400 Orsay (FR)**
Inventeur : **Colas, Claudine**
**16 rue Michelet**
**F-34130 Mauguio (FR)**
Inventeur : **Maire, Gérard**
**14, rue Perrey**
**F-31400 Toulouse (FR)**

(74) Mandataire : **Gillard, Marie-Louise et al**
**Cabinet Beau de Loménie 158, rue de l'Université**
**F-75340 Paris Cédex 07 (FR)**

EP 0 392 913 B1

Il est rappelé que : Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).

## Description

Le brevet français n° 2 387 229 a pour objet une famille de dipyrido [4,3-b] [3,4-f] indoles présentant d'intéressantes propriétés antitumorales.

Parmi les composés de cette famille, le (diéthylamino-3 propyl)-amino-1 méthyl-5 dipyrido [4,3-b] [3,4-f] indole de formule

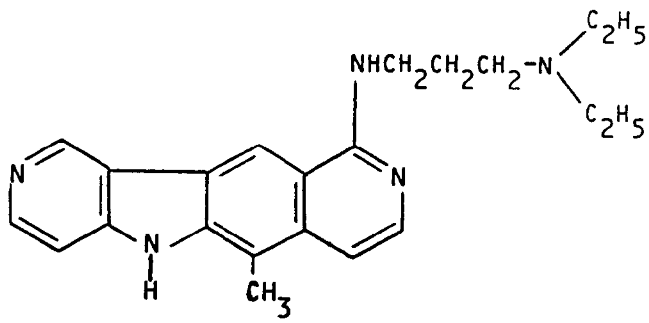

également dénommé [[(diéthylamino)-3 propyl] amino] -10 méthyl-6 5H-pyrido [3', 4' : 4,5] pyrrolo [2,3-g] isoquinoléine est bien connu dans la littérature scientifique sous le code "BD40" et possède maintenant la dénomination commune internationale "PAZELLIPTINE". Ce composé possède des propriétés antitumorales spécialement intéressantes qui ont conduit à des expérimentations chez l'animal et chez l'homme en tant que médicament.

Bien qu'il soit mentionné d'une façon générale que les dipyrido [4,3-b] [3,4-f] indoles objet du brevet français n° 2 387 229 peuvent donner des sels avec les acides pharmaceutiquement acceptables, aucun sel du BD40 n'y a été décrit.

Toutefois, il apparaît dans un article publié dans Journal of Liquid Chromatography, 1983, 6 (5) 939-949 que le BD40 a été utilisé sous forme de trimaléate.

Les essais effectués par la demanderesse ont mis en évidence que ce trimaléate n'était pas stable et qu'il se dégradait rapidement aussi bien sous forme de produit en vrac que sous forme de compositions pharmaceutiques.

La présente invention a donc pour objet un nouveau sel du BD40 qui présente une bonne stabilité et permet la préparation aisée de compositions pharmaceutiques stables.

La présente invention concerne en tant que produit nouveau le trichlorhydrate de (diéthylamino-3 propyl)-amino-1 méthyl-5 dipyrido [4,3-b] [3,4-f] indole ou un de ses solvates.

La stabilité de ce sel a été étudiée en comparaison avec le trimaléate du BD40.

Sur la matière première, à l'état solide ou en solution, l'étude de stabilité a été effectuée dans des conditions de vieillissement accéléré.

Les résultats obtenus après 20 jours sont réunis dans le tableau suivant.

Dans chaque cas sont indiquées les conditions opératoires utilisées. Les résultats sont indiqués sous la forme de pourcentage de produit non dégradé.

| | Trimaléate | Trichlorhydrate |
|---|---|---|
| matière première solide 80°C - 85 % d'humidité relative | 69,3 | 99,1 |
| solution aqueuse à 100 mg/ml 100°C à pH 3,4 | 40,9 | 92,7 |
| solution eau/méthanol 70/30 à 100 mg/ml - 100°C | 51,9 | 96,0 |
| solution aqueuse sous lumière intense 90000 lux | 1,0 | 85,6 |

De même, la stabilité a été comparée sur des préformulations galéniques, à savoir des lyophilisats obtenus à partir de solutions de trimaléate et de trichlorhydrate de BD40 en présence ou non de mannitol.

Avec le trimaléate, on constate dans tous les cas une dégradation rapide du principe actif pouvant atteindre 20 à 30 % en 1 mois à 50°C ou 3 mois à 37°C.

A l'inverse, un lyophilisat contenant le trichlorhydrate correspondant à 100 mg de base et 50 mg de mannitol ne présente pas d'impureté de dégradation après 1 an à 37°C.

Il existe donc une très grande différence de stabilité entre les deux sels du BD40.

Le trimaléate se dégrade rapidement alors que le trichlorhydrate est très stable.

Le trichlorhydrate de BD40 peut être préparé selon les méthodes habituelles de formation des sels et notamment par réaction de la base en phase aqueuse avec une solution aqueuse d'acide chlorhydrique. Après purification par action de charbon actif, la solution est lyophilisée. Le trichlorhydrate de BD40 peut être par exemple monohydraté.

Enfin, les activités pharmacologiques et toxicologiques des deux sels ont été comparées entre elles et à celle du BD40. Rapportées à une base molaire, ces activités n'ont pas fait apparaître de différence notable entre les deux sels et le BD40.

Grâce à sa stabilité, le produit de la présente invention peut être utilisé pour la préparation de compositions pharmaceutiques selon les techniques habituelles. Une telle utilisation constitue un autre objet de la présente invention.

L'exemple suivant illustre l'invention.

## EXEMPLE

Préparation du trichlorhydrate de (diéthylamino-3 propyl)-amino-1 méthyl-5 dipyrido [4,3-b] [3,4-f] indole.

A une solution de 1 g de BD40 base dans 10 ml d'eau déionisée, on ajoute sous agitation 0,9 g d'une solution aqueuse d'acide chlorhydrique à 37 %. Après 30 min d'agitation à 20-25°C, on décolore la solution par addition de 0,2 g de charbon actif et agitation pendant 30 min. Après filtration, on lyophilise la solution pendant 36 h avec une température, en fin d'opération, d'environ 25°C. On obtient ainsi 1,24 g (95 %) de trichlorhydrate de BD40 sous forme d'une poudre jaune-crème ; P.F. > 260°C.

Le titre par chromatographie en phase liquide est supérieur à 98 % sur produit sec.

Teneur en chlore ionique : 22,7 % (théorie 22,6 %)

Teneur en eau (Karl-Fischer) : 5,9 %

UV : lambda-max à 365, 348, 295 et 255 nm.

## Revendications

1. Le trichlorhydrate de (diéthylamino-3 propyl)-amino-1 méthyl-5 dipyrido [4,3-b] [3,4-f] indole ou un de ses solvates.

2. Procédé pour la préparation d'un composé selon la revendication 1, caractérisé en ce qu'on neutralise la base libre en phase aqueuse par une solution aqueuse d'acide chlorhydrique et on isole le produit par lyophilisation.

3. Utilisation d'un composé selon la revendication 1 pour la préparation de compositions pharmaceutiques.

## Patentansprüche

1. (3-Diethylaminopropyl)-1-amino-5-methyldipyrido[4,3-b][3,4-f]indoltrihydrochlorid oder eines seiner Solvate.

2. Verfahren zur Herstellung einer Verbindung nach Anspruch 1, dadurch gekennzeichnet, daß man die freie Base in wäßriger Phase durch eine wäßrige Salzsäurelösung neutralisiert und das Produkt durch Gefriertrocknen isoliert.

3. Verwendung einer Verbindung nach Anspruch 1 zur Herstellung von Arzneimitteln.

## Claims

1. 1-(3-Diethylaminopropyl)amino-5-methyldipyrido [4,3-b]-[3,4-f]indole trihydrochloride or one of its solvates.

2. A method of preparing a compound according to claim 1, characterized in that it comprises neutralizing the free base in the aqueous phase with an aqueous solution of hydrochloric acid and isolating the product by lyophilization.

3. Use of a compound according to claim 1 for the preparation of pharmaceutical compositions.